# EUROPEAN PATENT APPLICATION

(11) **EP 3 480 289 A1**
(43) Date of publication of application: **08.05.2019**
(21) Application number: 17820364.2
(22) Date of filing: 03.07.2017
(51) Int. Cl.: C12M 1/00, C12N 1/04

(54) **DEVICE FOR THAWING PRESERVED VITRIFIED GERM CELLS AND THAWING METHOD**

(30) Priority: 01.07.2016 JP 2016132037; 30.06.2017 JP 2017129765
(71) Applicant: Hiroshima Prefecture, Hiroshima-shi, Hiroshima 730-8511 (JP); Misawa Medical Industry Co., Ltd., Kasama-shi, Ibaraki 309-1717 (JP)
(72) Inventor: HIDAKA, Takemasa, Shobara-shi Hiroshima 727-0023 (JP); MORIMOTO, Kazuhide, Shobara-shi Hiroshima 727-0023 (JP); FUKUMOTO, Yutaka, Shobara-shi Hiroshima 727-0023 (JP); IMAI, Akira, Shobara-shi Hiroshima 727-0023 (JP); OGATA, Yasuhiro, Shobara-shi Hiroshima 727-0023 (JP); YAMAMOTO, Yuusuke, Shobara-shi Hiroshima 727-0023 (JP); MISAWA, Hiroyasu, Kasama-shi Ibaraki 309-1717 (JP)
(74) Representative: Bittner, Thomas L.
(86) International application number: PCT/JP2017/024407
(87) International publication number: WO 2018/004010

(57) **Abstract**

In order to provide a device for thawing a preserved vitrified germ cell and a method for thawing the preserved vitrified germ cell, both of which device and method are capable of simultaneously achieving a high conception rate and an easier transfer operation, a thawing device (1) includes a carrying device (8), a tube (5), and a cold storage device (6), the tube (5) including, inside the tube (5), (i) a germ cell containing region (10) containing the carrying device (8), and being covered by the cold storage device (6), and (ii) a thawing solution containing region (11) containing a thawing solution, and the carrying device (8) coming in contact with the thawing solution which has been melted.

## Description

The present invention relates to a device for thawing a preserved vitrified germ cell and a method for thawing the preserved vitrified germ cell.

### Background

In the field of animal husbandry, techniques for transferring an embryo into a reproductive organ of a mammalian animal (embryo transfer technique) are well-known as techniques for increasing a target mammal. In order to obtain better embryo transfer techniques, current development of embryo transfer techniques puts a focus on, for example, a method of freezing and preserving an embryo and/or a method of thawing a frozen embryo,

One known example of embryo transfer techniques is a method which uses a preserved embryo which has been vitrified by rapidly cooling (preservation method using ultra-rapid vitrification). According to this method, for example, after an embryo is mixed with vitrification solution in a petri dish, the vitrification solution containing the embryo is placed on a planar device and immersed in liquid nitrogen, so that the embryo is rapidly cooled so as to be vitrified and preserved. When the embryo is subsequently used for embryo transfer, the planar device, on which that preserved vitrified embryo is placed, is immersed in thawing solution. This rapidly warms and thaws the preserved vitrified embryo. Such a series of operations in the preservation method using ultra-rapid vitrification needs to be carried out not outdoors but in a laboratory that provides a sterile environment.

As another known example of embryo transfer techniques, Patent Literatures 1 and 2 each disclose a method using a preserved vitrified embryo in a straw (in-straw vitrification method). According to this method, the preserved vitrified embryo is thawed in a straw and then directly transferred to a target mammalian animal from the straw.

### Citation List

### [Patent Literature]

[Patent Literature 1]
   Japanese Patent Application Publication *Tokukai* No. 2014-143950 (Publication date: August 14, 2014)
[Patent Literature 2]
   Japanese Patent Application Publication *Tokukai* No. 2014-184056 (Publication date: October 2, 2014)

### Summary

### Technical Problem

However, the above conventional technique has a problem in that it is not possible to simultaneously achieve a high conception rate and an easy transfer operation.

For example, the preservation method using ultra-rapid vitrification makes it possible to increase the conception rate on one hand, but on the other, makes the transfer operation troublesome because a series of operations needs to be carried out not outdoors but in a laboratory that provides a sterile environment.

Meanwhile, the in-straw vitrification method can achieve an easier transfer operation on one hand, but on the other, has problems in that (i) a troublesome operation is necessary for vitrifying and preserving an embryo and storing the embryo in a straw and (ii) a survival rate of the embryo and the conception rate are low.

The present invention is attained in view of the above problems. An object of the present invention is to provide a device for thawing a preserved vitrified germ cell and a method for thawing the preserved vitrified germ cell, both of which device and method are capable of simultaneously achieving a high conception rate and an easier transfer operation.

### Solution to Problem

When a germ cell is vitrified and preserved or when a preserved vitrified germ cell is thawed, the germ cell suffers two kinds of damage. This consequently decreases a conception rate at the time when the germ cell is transferred. One kind of the damage to the germ cell is caused by a crack in ice crystal at a temperature of approximately -130°C (fracture damage). The other kind of the damage to the germ cell is caused by formation of an ice crystal at a temperature of approximately -80°C after devitrification.

As a result of diligent studies in light of the above problems, the inventors of the present invention have found the following. That is, while the temperature of a vitrified germ cell placed in a tube is prevented from changing and kept at a low temperature, the state of thawing solution in the tube is changed to a state suitable for thawing the germ cell in the tube (for example, the thawing solution is changed from a frozen state to a melted state). Thereafter, the thawing solution and the germ cell are immediately brought into contact with each other. This makes it possible not only to achieve a high conception rate by reducing damage to the germ cell but also to make a transfer operation easier by directly transferring, to an animal, the germ cell from the tube which contains the germ cell and which is mounted to a germ cell transfer device. On the basis of these findings, the inventors have accomplished the present invention.

In order to solve the above problems, a device for thawing a preserved vitrified germ cell in accordance with an embodiment of the present invention includes: a carrying device on which the preserved vitrified germ cell is placed; a tube in which the carrying device is contained; and a cold storage device provided on an outer periphery of the tube, the tube including, inside the tube: (i) a germ cell containing region containing the carrying device, and being filled with gas and covered by the cold storage device; and (ii) a thawing solution containing region containing a thawing solution for thawing the preserved vitrified germ cell, and the carrying device coming in contact with the thawing solution in a melted state.

In order to solve the above problems, a method for thawing a preserved vitrified germ cell in accordance with an embodiment of the present invention includes the steps of: (a) melting a thawing solution in a frozen state in a tube, the thawing solution being used for thawing the preserved vitrified germ cell, the thawing solution being melted by warming only a thawing solution containing region between (i) a germ cell containing region and (ii) the thawing solution containing region which are provided inside the tube, (i) the germ cell containing region containing a carrying device on which the preserved vitrified germ cell is placed, and being filled with gas and covered by a cold storage device provided on a periphery of the tube, and (ii) the thawing solution containing region containing the thawing solution in the frozen state; and (b) bringing the carrying device into contact with the thawing solution which has been melted.

### Advantageous Effects of Invention

An embodiment of the present invention makes it possible to avoid various kinds of damage (e.g., fracture damage and/or damage which is caused by formation of an ice crystal after devitrification) to a preserved vitrified germ cell and achieve a high survival rate when the preserved vitrified germ cell is thawed.

Further, an embodiment of the present invention makes it possible to significantly increase a conception rate of a livestock animal because a preserved vitrified germ cell can be thawed at a high survival rate.

Furthermore, an embodiment of the present invention makes it possible to easily prepare a thawed germ cell at a field site on a farm or the like, because a germ cell can be thawed without use of any special equipment (e.g., sterile room).

In addition, an embodiment of the present invention makes it possible to (i) mount a device for thawing to a germ cell transfer device, (ii) then insert the germ cell transfer device into a reproductive organ which is a target for germ cell transfer and (ii) transfer a thawed germ cell from the device for thawing to the reproductive organ. In other words, it is not necessary to transfer, into a separate tube or the like, a germ cell thawed by the device for thawing, and the device for thawing, which device contains the germ cell, can be mounted to the germ cell transfer device. Then, it is possible to insert the germ cell transfer device into a reproductive organ which is a target for germ cell transfer and then to transfer the germ cell into the reproductive organ from the device for thawing.

For a current freezing method which allows for direct transfer, an expensive programmed freezer (which costs approximately one million yen) is required. This increases cost and requires an electrical source for stably causing the programmed freezer to operate. In contrast, an embodiment of the present invention can decrease the cost and makes it possible to vitrify a germ cell at a site where an electrical source as above is absent.

In an embodiment of the present invention, a portion in which a vitrified germ cell is contained is kept cold by a cold storage device. Therefore, the vitrified germ cell is not easily influenced by an external temperature, even if the vitrified germ cell is exposed outside by error when a vitrified germ cell which has been sold is transferred from one liquid nitrogen container to another. Therefore, it is possible to keep survivability and preservability of the germ cell high.

### Brief Description of Drawings

- Fig. 1: is a view schematically illustrating an embodiment of a thawing device for a preserved vitrified germ cell in accordance with an embodiment of the present invention.
- Fig. 2: is a view schematically illustrating an embodiment of a thawing device for a preserved vitrified germ cell in accordance with an embodiment of the present invention.
- Fig. 3: is a view schematically illustrating an embodiment of a thawing device for a preserved vitrified germ cell in accordance with an embodiment of the present invention.
- Fig. 4: is a view schematically illustrating an embodiment of a cold storage device which is provided in a thawing device for a preserved vitrified germ cell in accordance with an embodiment of the present invention.
- Fig. 5: is a view schematically illustrating an embodiment of a partition wall which is provided in a thawing device for a preserved vitrified germ cell in accordance with an embodiment of the present invention.
- Fig. 6: is a graph showing temperature changes at various parts inside a thawing device in an Example of the present invention.
- Fig. 7: is a graph showing a temperature change in a germ cell containing region in each of cases where cold storage devices of various shapes were used in an Example of the present invention.
- Fig. 8: is a graph showing a temperature change in a germ cell containing region, in each of cases where cold storage devices made of various materials were used in an Example of the present invention.
- Fig. 9: is a graph showing temperature changes at various positions in a germ cell containing region which is covered by a cold storage device, in an Example of the present invention.
- Fig. 10: is a view schematically illustrating a Variation of an embodiment of a cold storage device provided in a thawing device for a preserved vitrified germ cell in accordance with an embodiment of the present invention.
- Fig. 11: is a graph showing a temperature change in a germ cell containing region, in each of cases where various cold storage devices were used in an Example of the present invention.

### Description of embodiments

The following description will discuss embodiments of the present invention. Note, however, that the present invention is not limited to the following embodiments, but can be altered by a skilled person in the art within the scope of the claims. The present invention also encompasses, in its technical scope, any embodiment or example derived by combining technical means disclosed in differing embodiments and/or examples. All academic and patent literatures listed herein are incorporated herein by reference. Any numerical range expressed as "A to B" herein means "not less than A and not more than B" unless otherwise stated.

### 1. Thawing device for preserved vitrified germ cell

### 1-1. Brief description of configuration of thawing device

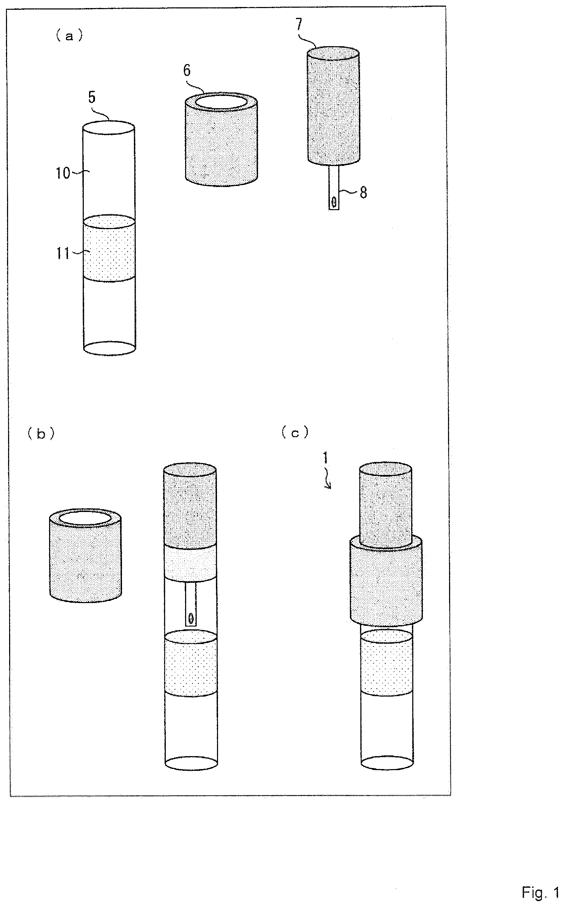

The following will briefly discuss a configuration of a thawing device for a preserved vitrified germ cell in accordance with an embodiment of the present invention, with reference to Fig. 1.

As illustrated in (a) to (c) of Fig. 1, a thawing device 1 includes, as constituents, a carrying device 8 on which a preserved vitrified germ cell is placed, a tube 5 in which the carrying device 8 is contained, and a cold storage device 6 which is provided on an outer periphery of the tube. Note that in (a) to (c) of Fig. 1, the carrying device 8 is connected with a force transmission device 7. The force transmission device 7 is not essential to the thawing device 1, and can be omitted as appropriate.

The tube 5 includes therein (i) a germ cell containing region 10 covered by a cold storage device 6, in which germ cell containing region 10 the carrying device 8 is contained and which germ cell containing region 10 is filled with gas (in other words, the germ cell containing region 10 is a space filled with gas, in which space the carrying device 8 is contained) and (ii) a thawing solution containing region 11 in which a thawing solution for thawing a preserved vitrified germ cell is contained (in other words, a thawing solution containing region 11 is a space configured to contain a thawing solution for thawing a preserved vitrified germ cell).

As illustrated in (b) of Fig. 1, in the thawing device 1, the carrying device 8 is contained inside the germ cell containing region 10 which is provide inside the tube 5. Further, as illustrated in (c) of Fig. 1, in the thawing device 1, the cold storage device 6 covers the outside of the germ cell containing region 10 in which the carrying device 8 is contained. In such a configuration, the carrying device 8 is covered by the cold storage device 6. This makes it possible to keep the germ cell, which is placed on the carrying device 8, in a low temperature environment for a long time.

As illustrated in (b) and (c) of Fig. 1, the carrying device 8 and the thawing solution contained in the thawing solution containing region 11 are initially provided so as not to come in contact with each other. Specifically, the carrying device 8 and the thawing solution contained in the thawing solution containing region 11 are initially arranged such that the carrying device 8 is not immersed in the thawing solution. Then, when the thawing device 1 is used (specifically when a preserved vitrified germ cell is to be thawed), the carrying device 8 is brought into contact with the thawing solution in a melted state. More specifically, when the thawing device 1 is used (specifically when a preserved vitrified germ cell is to be thawed), the carrying device 8 is immersed in the thawing solution in a melted state. Note that the expression that "(A and B) come in contact with each other" herein also means "(A) is immersed (into B)". The above configuration causes the germ cell in a low temperature environment to immediately come in contact with the thawing solution. This makes it possible to avoid various kinds of damage (e.g., fracture damage and/or damage which is caused by formation of an ice crystal after devitrification) to a preserved vitrified germ cell and achieve a high survival rate when the preserved vitrified germ cell is thawed.

Note that the thawing device in accordance with an embodiment of the invention of the present application encompasses both a thawing device in which a germ cell is placed on the carrying device and a thawing device in which no germ cell is placed on the carrying device.

The thawing device 1 can be used not only as a "thawing device" for thawing a preserved vitrified germ cell but also as a "vitrification tool" for vitrification of a germ cell and/or as "preservation tool" for preservation of a vitrified germ cell.

For example, in a case where the thawing device 1 is used as a "vitrification tool", a germ cell balanced with vitrification solution is placed on the carrying device 8 and this carrying device 8 is directly immersed into liquid nitrogen, so that the germ cell is vitrified. Thereafter, the carrying device 8 is inserted and joined to the tube 5 in liquid nitrogen. At this time, the cold storage device 6 has been attached to the tube 5, and the tube 5 contains the thawing solution, which has been drawn into the tube 5 and frozen in the tube 5 in advance. The thawing device 1 as illustrated in Fig. 1 is thus produced. Alternatively, in a case where the thawing device 1 is used as a "vitrification tool", a germ cell balanced with vitrification solution is placed on the carrying device 8 and this carrying device 8 is inserted and joined to the tube 5, which contains the thawing solution having been drawn into the tube 5 and to which the cold storage device 6 has been attached. The thawing device 1 as illustrated in Fig. 1 is thus produced. Then, the germ cell can be vitrified by entirely immersing this thawing device 1 in liquid nitrogen.

In a case where the thawing device 1 is used as a "preservation tool", the thawing device 1 having been immersed in liquid nitrogen is kept in a low temperature environment (e.g., in liquid nitrogen), so that a vitrified germ cell can be preserved.

In a case where the thawing device 1 is used for transfer of a germ cell, the germ cell, which has been placed on the carrying device 8 and vitrified, is brought into contact with the thawing solution in a melted state and thawed. Then, the thawing device 1 is attached to a separate germ cell transfer device. Subsequently, the germ cell transfer device including the thawing device is inserted into a reproductive organ of a target animal and the germ cell is ejected into the reproductive organ. As a result, the germ cell can be transferred. Note that the germ cell transfer device is not particularly limited in configuration, and can be any germ cell transfer device available in the market.

The following will discuss further details of each configuration of the thawing device 1.

### 1-2. Carrying device

The carrying device is a configuration for placing thereon a preserved vitrified germ cell. Note that on the carrying device which is provided in the thawing device in accordance with an embodiment of the present invention, either a preserved vitrified germ cell or a raw germ cell prior to preservation by vitrification (e.g., a mixture of liquid-state vitrification solution and a germ cell) may have been placed, or neither may have been placed.

In a case where a raw germ ceil prior to preservation by vitrification has been placed on the carrying device, for example, the carrying device on which a preserved vitrified germ cell is placed can be provided by immersing, into liquid nitrogen or the like, the thawing device which includes the carrying device.

In a case where neither a preserved vitrified germ cell nor a raw germ cell prior to preservation by vitrification has not yet been placed on the carrying device, for example, the carrying device on which a preserved vitrified germ cell is placed can be provided by (i) separately preparing a preserved vitrified germ cell and (ii) placing the preserved vitrified germ cell on the carrying device.

A material of the carrying device is not limited to a particular one. The material is preferably a material which allows the temperature of the preserved vitrified germ cell to easily change in a case where the preserved vitrified germ cell is thawed. Examples of such a material encompass: polyolefins (e.g., polyethylene, ultra-high molecular weight polyethylene, polypropylene, an ethylene-propylene copolymer, and an ethylene-vinyl acetate copolymer), polyesters (e.g., polyethylene terephthalate and polybutylene terephthalate), styrene-based resins (e.g., polystyrene, a methacrylate-styrene copolymer, and a methacrylate-butylene-styrene copolymer), polyamides (e.g., nylon 6, and nylon 66), polysulfone resins, fluorine resins, vinyl chloride, nylon, and polyimide.

Alternatively, the carrying device can be made of a metal or the like and configured to have a net-like structure (mesh). The metal is not limited to a particular kind, and can be, for example, stainless steel, aluminum, and copper. In a case where the carrying device is made of a metal or the like and configured to have a net-like structure, an operation under transmitted light is possible. This makes it easy to see and handle a germ cell. Further, a mesh made of ultrafine wire is more preferable than a metal plate, since such a mesh has a lower heat capacity than a metal plate. Alternately, the carrying device can be made of a punched metal.

The germ cell is not particularly limited. The germ cell can be a fertilized egg (e.g., a fertilized egg(s) collected from a female reproductive organ or fertilized egg prepared by in vitro fertilization), an embryo prepared by microfertilization, an embryo prepared by nuclear transplantation, or a sperm.

The origin of the germ cell is not particularly limited. The germ cell can be, for example, a human germ cell or a non-human germ cell (e.g., a germ cell of a non-human vertebrate animal or a germ cell of a non-human mammalian animal). More specifically, the germ cell can be any of germ cells of cows, pigs, sheep, goats, horses and humans. Certainly, an embodiment of the present invention is not limited to these germ cells.

In a case where a preserved vitrified germ cell is to be prepared, a germ cell is balanced with vitrification solution and then the germ cell is cooled with use of liquid nitrogen or the like.

The vitrification solution is not particularly limited and can be a well-known vitrification solution. For example, a usable vitrification solution can be obtained by dissolving, in phosphate buffered saline (PBS) or the like, one or a combination of two or more of glycerol, ethylene glycol, dimethyl sulfoxide (DMSO), propylene glycol, butanediol and the like. Further, it is possible to add as appropriate, to the vitrification solution thus obtained, sucrose, glucose, bovine serum albumin, trehalose, Percoll, polyethylene glycol, polyvinyl pyrrolidone, or Ficoll serum.

The vitrification solution can be, for example, a Medium-199 solution which contains ethylene glycol, dimethyl sulfoxide (DMSO), sucrose and bovine serum albumin. For example, respective concentrations of ethylene glycol, dimethyl sulfoxide (DMSO), sucrose and bovine serum albumin can be set such that the concentration of ethylene glycol is approximately 15% by weight, the concentration of dimethyl sulfoxide (DMSO) is approximately 15% by weight, the concentration of sucrose is approximately 0.5 M, and the concentration of bovine serum albumin is approximately 0.4% by weight.

An embodiment of the present invention can be configured such that: (i) the carrying device moves toward the thawing solution in a melted state, so that the carrying device and the thawing solution come in contact with each other; (ii) the thawing solution in a melted state moves toward the carrying device, so that the carrying device and the thawing solution come in contact with each other; or (iii) the carrying device moves toward the thawing solution in a melted state while the thawing solution moves toward the carrying device, so that the carrying device and the thawing solution come in contact with each other. The configuration (i) is preferable from the viewpoint of immediately and unfailingly bringing a germ cell, which is kept in a low temperature environment, into contact with the thawing solution in a state suitable for thawing the germ cell.

The carrying device may be caused to move in a pattern that is not particularly limited. The following will discuss moving pattern examples of the carrying device.

### A. Moving pattern 1

The carrying device can be pushed toward the thawing solution in a melted state, by moving the force transmission device connected with the carrying device.

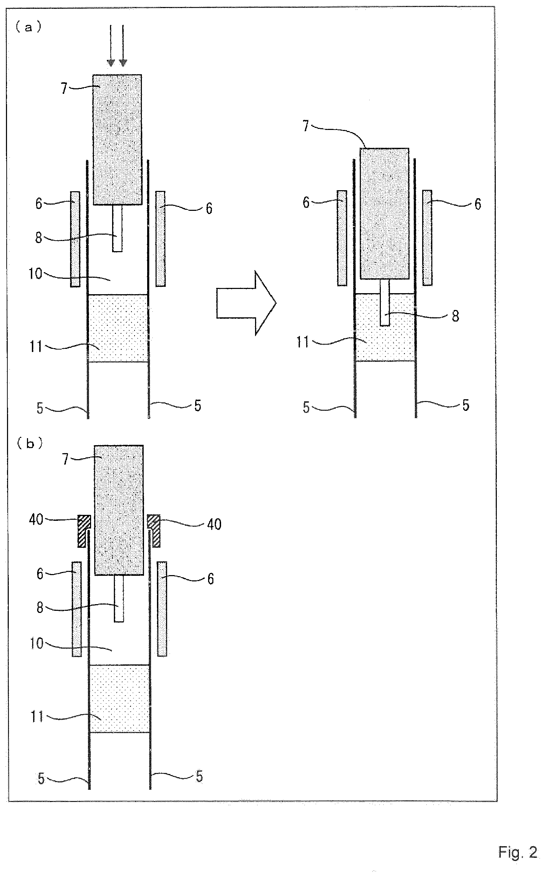

Fig. 2 illustrates one example of a longitudinal cross section of the present thawing device.

As illustrated on the left of a white arrow in (a) of Fig. 2, in the present thawing device, the force transmission device 7 is connected with the carrying device 8. The carrying device 8 is provided inside the germ cell containing region 10 which is covered by the cold storage device 6. In this case, the thawing solution containing region 11 is provided so as to be adjacent to the germ cell containing region 10. When force is applied to the force transmission device 7 in a direction shown by black arrows, a composite body constituted by the force transmission device 7 and the carrying device 8 moves inside the tube 5 toward the thawing solution containing region 11.

When the composite body moves inside the tube 5 toward the thawing solution containing region 11, the carrying device 8 and the thawing solution containing region 11 come in contact with each other as illustrated on the right of the white arrow in (a) of Fig. 2. As a result, a germ cell placed on the carrying device 8 is thawed.

The force transmission device 7 is not particularly limited in configuration and shape. The force transmission device 7 is preferably a bar which is made of the same material as the carrying device 8, which can be inserted into the tube 5, and at least a portion of which has a shape that fits the shape of an inner wall of the tube 5. For example, in a case where the tube 5 has a cylindrical shape, the force transmission device 7 preferably has a columnar shape.

In a case where the force transmission device 7 is made of the same material as the carrying device 8, a germ cell placed on the carrying device 8 can be kept in a low temperature environment for a long time.

Meanwhile, in a case where the force transmission device 7 is a bar that can be inserted into the tube 5 and at least a portion of which has a shape that fits the inner wall of the tube 5, the composite body constituted by the force transmission device 7 and the carrying device 8 can smoothly move inside the tube 5 toward the thawing solution containing region 11 when force is applied to the force transmission device 7.

Further, as illustrated in (b) of Fig. 2, a stopper device 40 can be provided at an end portion of the tube 5. The stopper device 40 is arranged to be in contact with the force transmission device 7 and causes friction with the force transmission device 7. This temporarily stops move of the composite body constituted by the force transmission device 7 and the carrying device 8, and allows the composite body to temporarily stay at a desired position. The shape of the stopper device 40 is not particularly limited, and can be a shape covering the force transmission device 7.

The above-described configuration allows the composite body constituted by the force transmission device 7 and the carrying device 8 to move inside the tube 5 in a case where force in a direction indicated by the black arrows in (a) of Fig. 2 is applied to the force transmission device 7, and in contrast, to stay at a desired position in the tube 5 in a case where no force is applied to the force transmission device 7 as illustrated on the right of the white arrow in (a) of Fig. 2. This makes it possible to keep the carrying device 8 at a desired position in the germ cell containing region 10 or in the thawing solution containing region 11 in the tube 5.

### B. Moving pattern 2

The carrying device can be caused to fall toward the thawing solution in a melted state, by providing the thawing device such that the germ cell containing region is placed above the thawing solution containing region.

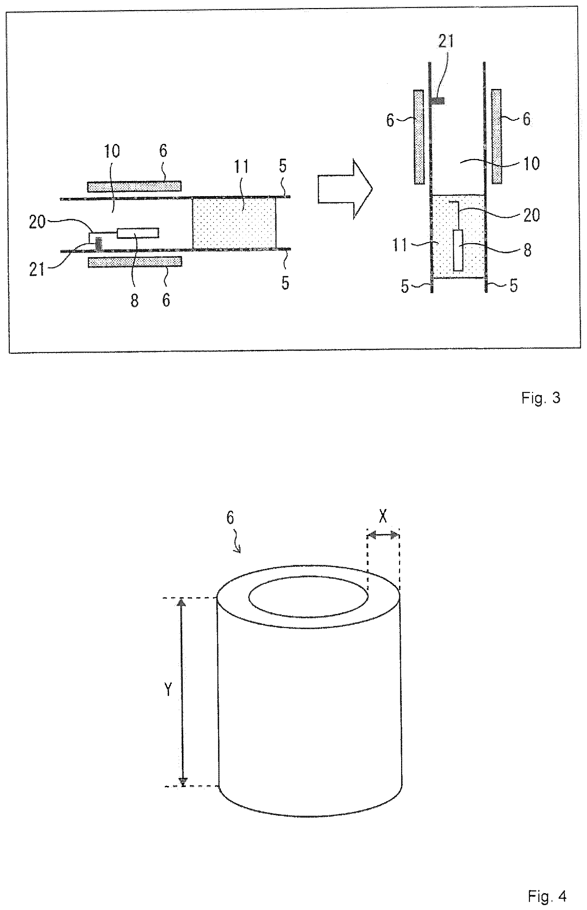

Fig. 3 illustrates one example of a longitudinal cross section of the present thawing device.

As illustrated on the left of a white arrow in (a) of Fig. 3, in the present thawing device, the carrying device 8 is provided inside the germ cell containing region 10 which is covered by the cold storage device 6. In this configuration, the thawing solution containing region 11 is provided so as to be adjacent to the germ cell containing region 10.

As illustrated on the right of the white arrow in Fig. 3, in a case where the thawing device is arranged such that the germ cell containing region 10 is above the thawing solution containing region 11 (for example, the thawing device is caused to stand), the carrying device 8 falls toward the thawing solution in the thawing solution containing region 11. Consequently, a germ cell placed on the carrying device 8 is thawed.

In this configuration, the carrying device 8 can be provided with a first locking device 20, while the tube 5 can be provided with a second locking device 21 on an inner wall of the tube 5. Then, the first locking device 20 and the second locking device 21 can be locked together in a detachable manner. More specifically, the present thawing device can be configured such that: in a case where the thawing device is laid as illustrated on the left of the white arrow in Fig. 3, the first locking device 20 and the second locking device 21 are locked together; and in contrast, in a case where the thawing device is caused to stand as illustrated on the right of the white arrow in Fig. 3, the first locking device 20 and the second locking device 21 are unlocked. In the above configuration, in a case where the present thawing device is laid, the carrying device 8 can be stably placed in the germ cell containing region 10 covered by the cold storage device 6. In contrast, in a case where the carrying device 8 is caused to stand, the carrying device 8 can be easily moved toward the thawing solution containing region 11.

Note that in a case where the inner wall itself of the tube 5 has a function of the second locking device 21 (e.g., in a case where the surface of the inner wall is uneven), the second locking device 21 can be omitted while only the first locking device 20 is provided. In contrast, in a case where the carrying device 8 itself has a function of the first locking device 20 (e.g., in a case where the carrying device 8 has a bending shape), the first locking device 20 can be omitted while only the second locking device 21 is provided.

The first locking device 20 can be, for example, a projecting structure which is made of the same material as the tube 5. On the other hand, the second locking device 21 can be, for example, a depressed structure which is made of the same material as the carrying device 8 and which is capable of receiving the projecting structure. Alternatively, the second locking device 21 can be an annular structure on which the projecting structure can be hung. Needless to say, the shape of the first locking device 20 and the shape of the second locking device 21 are by no means limited to the above-described shapes.

The present thawing device certainly can be configured to include neither the first locking device 20 nor the second locking device 21.

### 1-3. Tube

The present thawing device includes a tube in which the above-described carrying device is contained.

A material of the tube is not particularly limited, provided that the tube is resistant to a low-temperature state due to liquid nitrogen etc. Examples of the material of the tube encompass: polyolefins (e.g., polyethylene, ultra-high molecular weight polyethylene, polypropylene, an ethylene-propylene copolymer, and an ethylene-vinyl acetate copolymer), polyesters (e.g., polyethylene terephthalate and polybutylene terephthalate), styrene-based resins (e.g., polystyrene, a methacrylate-styrene copolymer, and a methacrylate-butylene-styrene copolymer), polyamides (e.g., nylon 6, and nylon 66), polysulfone resins, fluorine resins, vinyl chloride, and polyimide. Among these, vinyl chloride is preferable since a tube made of vinyl chloride has the following advantages: (i) the tube is transparent and therefore, the inside of the tube is visible; (ii) the tube hardly breaks even when the thawing solution inside the tube freezes and expands; and (iii) vinyl chloride itself as a material of the tube is non-cytotoxic.

The shape of the tube is not particularly limited, provided that the shape of the tube allows the tube to be covered by a cold storage device (described later). The tube can have, for example, a tubular shape (e.g., a polygonal columnar shape or a cylindrical shape).

With regard to the cross sectional shape of the tube, respective shapes of arbitrarily selected cross sections perpendicular to a direction in which the tube extends can be identical to each other or different from each other. More specifically, the tube may have a portion thicker than other portions which are thin.

In a case where the shape of the tube is tubular, the size of a space inside the tube is not particularly limited. For example, in a case where the shape of the tube is a cylindrical shape, the diameter of a circular space of a transverse cross section of the tube can be in the range of 1.8 mm to 2.2 mm or 2.8 mm to 3.2 mm. This configuration makes it possible to join the tube and an existing germ cell transfer device.

The tube includes, inside the tube, (i) a germ cell containing region, which contains the carrying device and is filled with gas and covered by the cold storage device, and (ii) a thawing solution containing region, which contains thawing solution for thawing the preserved vitrified germ cell.

The germ cell containing region is a portion of the tube. This region can be defined as a region covered by the cold storage device (described iater). The size and the shape of the germ cell containing region are not particularly limited, and can be set as appropriate in accordance with the shape and the size of the tube and the shape and the size of the cold storage device.

The thawing solution containing region is a portion of the tube. This region can be defined as a region occupied by the thawing solution. The size and the shape of the thawing solution containing region is not particularly limited, and can be set as appropriate in accordance with the amount of the thawing solution.

The number of the thawing solution containing region(s) is not particularly limited. There can be a plurality of thawing solution containing regions. For example, the thawing solution containing regions can be formed such that: (i) a first thawing solution containing region is formed in adjacent to the germ cell containing region; (ii) a first buffer region filled with gas is formed adjacent to the first thawing solution containing region; (iii) a second thawing solution containing region is formed adjacent to the first buffer region; (iv) a second buffer region filled with gas is formed adjacent to the second thawing solution containing region; and (v) a third thawing solution containing region is formed adjacent to the second buffer region (one example arrangement in a case where three thawing solution containing regions are formed).

The gas filling the germ cell containing region is not particularly limited, and is preferably, for example, sterile gas which is non-toxic to germ cells. Such gas can be, for example, gas sterilized by a filter.

The thawing solution is not particularly limited and can be a well-known thawing solution. For example, a usable thawing solution can be obtained by adding, to phosphate buffered saline (PBS) or the like, sucrose so as to have a sucrose concentration of 0.1 M to 0.5 M. Further, to the thawing solution thus obtained, serum can be added so that the concentration of the serum will be in the range of 10% to 30%,

### 1-4. Cold storage device

The present cold storage device is provided on an outer periphery of the tube so as to cover the germ cell containing region. The cold storage device prevents a change in temperature of the germ cell containing region, more specifically, a change in temperature of a germ cell which is contained in the germ cell containing region. This allows the germ cell to be kept in a low temperature environment for a long time.

A material of the cold storage device is not particularly limited. The material can be, for example, a material which has a heat capacity of preferably not less than 1300 J/K, more preferably not less than 1500 J/K, even more preferably not less than 1700 J/K, even more preferably not less than 2000 J/K, even more preferably not less than 2500 J/K, even more preferably not less than 3000 J/K, most preferably not less than 3500 J/K. The above configuration allows a germ cell, which is placed on the carrying device, to be kept in a low temperature environment for a longer time.

Specific examples of the material of which the cold storage device is made encompass silicon rubber, epoxy resin, copper, stainless steel, aluminum, polyethylene, polypropylene, or polystyrene. Note that Table 1 below shows respective heat capacities of the above materials.

**Table 1**

| Name of material | Density (a) [g/cm³] | Specific heat capacity (b) [J/(kg·K)] | Heat capacity ((a) × (b)) [J/K] |
|---|---|---|---|
| Silicon rubber | 970 | 1.6 | 1552 |
| Epoxy resin | 1850 | 1.1 | 2035 |
| Copper | 8880 | 0.386 | 3427.68 |
| Stainless steel | 7920 | 0.499 | 3952.08 |
| Aluminum | 2.6986 | 900 | 2429.01 |
| Polyethylene | 0.92 | 2320 | 2134.4 |
| Polypropylene | 0.91 | 1920 | 1747.2 |
| Polystyrene | 1.04 | 1340 | 1393.6 |

The shape of the cold storage device is not particularly limited, provided that the cold storage device can partially cover the tube. The following will describe an example of the shape of the cold storage device, with reference to Fig. 4.

As illustrated in Fig. 4, for example, the cold storage device 6 can have a tubular shape which allows the tube to be inserted in the cold storage device 6. The cold storage device 6 of the tubular shape can have a tube wall thickness X of not less than 0.2 mm, preferably not less than 0.6 mm, most preferably not less than 10 mm in a cross section of the tubular shape in a direction perpendicular to a direction in which the cold storage device 6 of the tubular shape extends. This configuration makes it possible to keep, in a low temperature environment for a longer time, the germ cell on the carrying device (not illustrated in Fig. 4) which is placed in a space inside the cold storage device 6.

As illustrated in Fig. 4, for example, the cold storage device 6 can have a tubular shape which allows the tube to be inserted in the cold storage device 6. The cold storage device 6 of the tubular shape can have a length Y of not less than 6 mm, preferably not less than 20 mm, most preferably not less than 30 mm in a direction in which the cold storage device 6 of the tubular shape extends. In this configuration, the germ cell on the carrying device (not illustrated in Fig. 4) which is placed in the space inside the cold storage device 6 can be placed not less than 3 mm apart from respective ends of the cold storage device 6 (in other words, an upper surface and a lower surface of the cold storage device 6 illustrated in Fig. 4). As a result, this configuration makes it possible to keep, in a low temperature environment for a longer time, the germ cell on the carrying device (not illustrated in Fig. 4) which is placed in a space inside the cold storage device 6.

### 1-5. Partition wall

The present thawing device is provided with a partition wall outside the tube. The partition wall can separate an atmosphere A to which the cold storage device is exposed and an atmosphere B to which a portion of the tube is exposed, which portion of the tube is a portion where the thawing solution containing region is provided. Since the above configuration includes the partition wall, the germ cell on the carrying device which is covered by the cold storage device is less influenced by the atmosphere B (e.g., the temperature of the atmosphere B) even in a case where respective conditions of the atmosphere A and the atmosphere B are different from each other. As a result, the thawing solution can be changed to a state more suitable for thawing the germ cell while the germ cell is kept in a low temperature environment (e.g., the thawing solution which is frozen can be melted while the germ cell is kept in a low temperature environment).

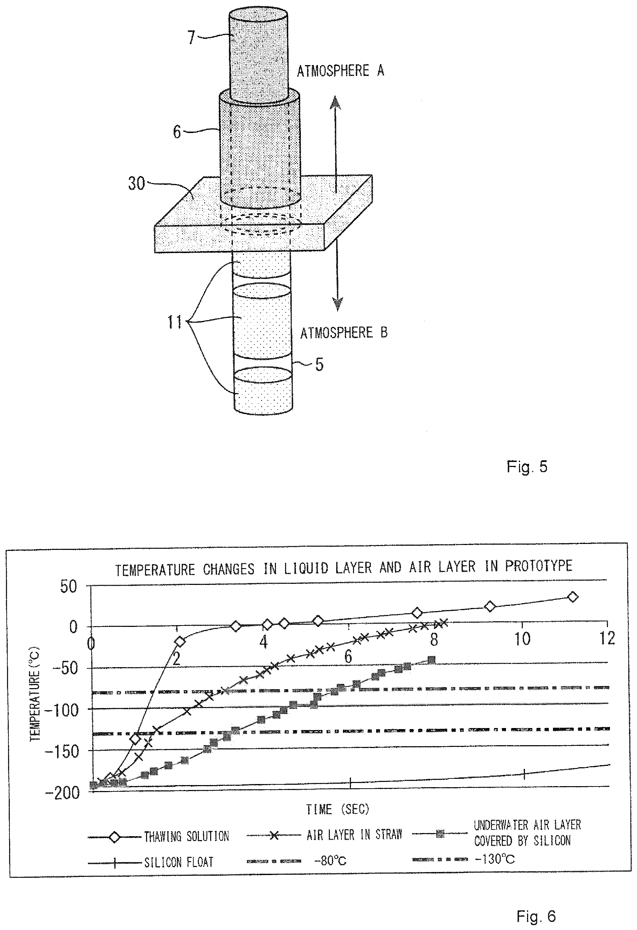

As illustrated in Fig. 5, for example, a partition wall 30 can be provided so as to abut on the cold storage device 6 outside the tube 5. Then, the atmosphere to which the present thawing device is exposed is separated into the atmosphere A and the atmosphere B, by the partition wall 30 which serves as a border between the atmosphere A and the atmosphere B.

The atmosphere A and the atmosphere B can be each an air layer or liquid layer. For example, the atmosphere A and the atmosphere B can be arranged such that (a) the atmosphere A is a low-temperature air layer while the atmosphere B is an air layer whose temperature is higher than that of the atmosphere A or (b) the atmosphere A is a low-temperature air layer while the atmosphere B is a liquid layer whose temperature is higher than that of the atmosphere A. The arrangement (a) is more preferable than the arrangement (b), from the viewpoint of more rapidly changing the thawing solution into a state more suitable for thawing a germ cell (e.g., changing the thawing solution from a frozen state to a melted state).

In the arrangement (a), the partition wall 30 can be, for example, a plate which gas cannot pass through. Note that a material, the size and the shape of the plate are not particularly limited.

In the arrangement (b), the partition wall (30) can be a float plate which itself is capable of floating on the liquid layer and which makes it possible to sink the thawing solution containing region 11 into the liquid layer while keeping the cold storage device 6 in the air layer. Note that a material, the size and the shape of the float plate are not particularly limited. Examples of the material constituting the float plate encompass expanded resin materials such as polyurethane, polyvinyl chloride, polypropylene, urethane-based sponge, synthetic rubber, and various elastomers.

Note that it is possible to use, in place of the float plate, a configuration in which a stand having a height equal to the depth of liquid in the liquid layer is sunk in the liquid layer and a plate having a hole is placed on the top of the stand. In this case, the thawing device is inserted into the hole and the thawing solution containing region is sunk into the liquid layer while the cold storage device is kept in the air layer. Further, in the above case, the stand can be made of stainless steel and the plate can be made of polystyrene or the like.

The size of the hole for insertion of the thawing device in the float plate or the plate is preferably smaller than the cold storage device and larger than the tube,

### 2. Variation of thawing device for preserved vitrified germ cell

In the present thawing device, a cold storage device 61 can have a space for retaining a refrigerant (e.g., liquid nitrogen). The cold storage device 61 can have a shape which includes a space for retaining a refrigerant, for example, between a wall of the cold storage device 61 and the tube 5. In the above configuration, the refrigerant is retained in the space when the thawing device cooled in the refrigerant is taken out of the refrigerant. Then, the refrigerant retained in the space makes it possible to keep an atmosphere in the germ cell containing region at a low temperature for a longer time, until a vitrified germ cell is immersed in the thawing solution in a melted state.

The cold storage device 61 can include a bottom portion 62 for blocking the space or both of a bottom portion 62 and a cover 63 for closing the space. Provision of the bottom portion 62 makes it possible to unfailingly retain the refrigerant in the space. On the other hand, provision of both of the bottom portion 62 and the cover 63 makes it possible to not only unfailingly retain the refrigerant in the space but also prevent loss of the refrigerant in the space due to, for example, vaporization or the like of the refrigerant.

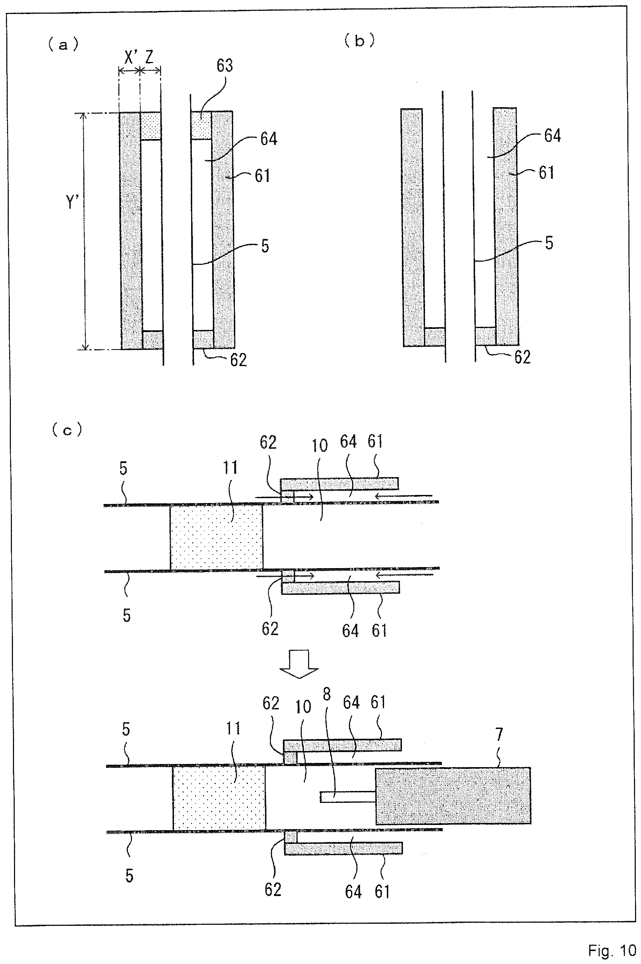

The following will describe an example of the cold storage device 61 with reference to (a) and (b) of Fig. 10. (a) and (b) of Fig. 10 are each a cross-sectional view of the cold storage device 61 attached to the tube 5. (a) of Fig. 10 illustrates the cold storage device 61 which is provided with both of the bottom portion 62 and the cover 63. (b) of Fig. 10 illustrates the cold storage device 61 which is provided with only the bottom portion 62. A material of the cold storage device 61 is not particularly limited. The cold storage device 61 can be made of a material which is described in [1-4. Cold storage device] and which has the heat capacity described in [1-4. Cold storage device].

As illustrated in (a) of Fig. 10, the cold storage device 61 can have a tubular shape which allows the tube to be inserted into the cold storage device61. The cold storage device 61 of the tubular shape can have a tube wall thickness X' of not less than 0.2 mm, preferably not less than 0.5 mm in a cross section of the tubular shape in a direction perpendicular to a direction in which the cold storage device 61 of the tubular shape extends. This configuration makes it possible to retain a large amount of refrigerant in a refrigerant retaining region 64 and also makes it possible to keep, in a low temperature environment for a longer time, a germ cell on the carrying device 8 which is placed in the germ cell containing region 10.

As illustrated in (a) of Fig. 10, the cold storage device 61 of the tubular shape can have a length Y' of not less than 6 mm, preferably not less than 20 mm, most preferably not less than 30 mm in a direction in which the cold storage device 61 of the tubular shape extends. In this configuration, the germ cell on the carrying device 8 which is placed in the germ cell containing region 10 can be placed not less than 3 mm apart from respective ends of the cold storage device 61 (in other words, an upper surface and a lower surface of the cold storage device 61 illustrated in (a) of Fig. 10). Further, the above configuration makes it possible to retain a larger amount of refrigerant in the refrigerant retaining region 64 and accordingly, makes it possible to keep the germ cell, which is placed on the carrying device 8, in a low temperature environment for a longer time.

As illustrated in (a) of Fig. 10, the width Z of the above-described space is not less than 0.5 mm and not more than 1.5 mm, preferably not less than 0.7 mm and not more than 1.2 mm. This configuration makes it possible to retain a larger amount of refrigerant in the refrigerant retaining region 64 and accordingly, makes it possible to keep the germ cell, which is placed on the carrying device 8, in a low temperature environment for a longer time. Additionally, the above configuration makes it possible to decrease an outer diameter of the cold storage device 61.

As illustrated in (a) of Fig. 10, the cold storage device 61 can have, at one end thereof, the bottom portion 62 for retaining the refrigerant. The bottom portion 62 is preferably made of a high-density material, from the viewpoint of retaining the refrigerant which has entered the refrigerant retaining region 64. The bottom portion 62 can be made of, for example, natural sponge, melamine sponge, silicon, or the like. In the above configuration, the bottom portion 62 can not only retain, in the refrigerant retaining region 64, the refrigerant which has entered the refrigerant retaining region 64, but also cause the refrigerant to enter the refrigerant retaining region 64.

As illustrated in (a) of Fig. 10, the cold storage device 61 can have, at the other end thereof, the cover 63 for covering the refrigerant. The material of the cover 63 can be similar to that constituting the bottom portion 62. Note however that the cover 63 is preferably made of a lower-density material as compared to the bottom portion 62, from the viewpoint of causing the refrigerant to easily enter the refrigerant retaining region 64.

The bottom portion 62 and the cover 63 can be provided so as to completely fill a gap between the tube 5 and the cold storage device 61 or to partially fill the gap between the tube 5 and the cold storage device 61.

The following will discuss a mechanism by which the refrigerant stays in the liquid nitrogen retaining region 64, with reference to (c) of Fig. 10.

After the thawing solution is drawn into the thawing solution containing region 11, the thawing device, to which the cold storage device 61 is attached, is placed in a refrigerant. Thereafter, the carrying device 8 on which a vitrified germ cell is placed is inserted into the tube 5 constituting the thawing device. Then, the thawing device is cooled in the refrigerant. At this time, the refrigerant enters the refrigerant retaining region 64 (arrows in (c) of Fig. 10).

Subsequently, the thawing device standing along a vertical direction is taken out of the refrigerant. At this time, the refrigerant having entered the refrigerant retaining region 64 is retained in the refrigerant retaining region 64 by the bottom portion 62. As a result, it is possible to keep, in a low temperature environment, a germ cell in the germ cell containing region 10 until the refrigerant in the refrigerant retaining region 64 is lost due to, for example, vaporization.

Note that (c) of Fig. 10 illustrates, as an example, the thawing device which does not include the cover 63. However, even in a case where the thawing device including the cover 63 is used, the refrigerant is similarly retained in the refrigerant retaining region 64. As described above, the density of the material of the cover 63 is lower than that of the bottom portion 62. Accordingly, when the thawing device is stored in the refrigerant, the refrigerant can pass through the cover 63 and be retained in the refrigerant retaining region 64.

Note that though (c) of Fig. 10 illustrates, as an example, a configuration using the carrying device 8 to which the force transmission device 7 is connected, the thawing device can be configured to include the first locking device 20 and the second locking device 21.

### 3. Method for thawing preserved vitrified germ cell

A method for thawing a preserved vitrified germ cell in accordance with an embodiment of the present invention includes the step of: (a) melting a thawing solution in a frozen state in a tube, the thawing solution being used for thawing the preserved vitrified germ cell, the thawing solution being melted by warming only a thawing solution containing region between (i) a germ cell containing region and (ii) the thawing solution containing region which are provided inside the tube, (i) the germ cell containing region containing a carrying device on which the preserved vitrified germ cell is placed, and being filled with gas and covered by a cold storage device provided on a periphery of the tube, and (ii) the thawing solution containing region containing the thawing solution in the frozen state; and (b) bringing the carrying device into contact with the thawing solution which has been melted.

With regard to configurations of the present method for thawing a preserved vitrified germ cell, descriptions on configurations described earlier will be omitted here.

In a case where the thawing solution which is frozen is to be melted by heating only the thawing solution containing region, it is preferable to warm, for example, a portion of the tube corresponding to the thawing solution containing region, at a temperature of 20°C to 38°C, preferably at a temperature of 25°C to 30°C. In the above configuration, the thawing solution which is frozen can be rapidly melted while a germ cell placed on the carrying device is kept in a low temperature environment for a long time.

In a case where the carrying device is to be brought into contact with the thawing solution which has been melted, for example, the temperature of the thawing solution is, though not particularly limited, preferably in the range of 15°C to 38°C and more preferably in the range of 25°C to 30°C. The above configuration makes it possible to avoid various kinds of damage (e.g., fracture damage and/or damage which is caused by formation of an ice crystal after devitrification) to a preserved vitrified germ cell and achieve a high survival rate when the preserved vitrified germ cell is thawed.

An embodiment of the present invention can be configured as below.

In order to solve the above problems, a device for thawing a preserved vitrified germ cell in accordance with an embodiment of the present invention includes: a carrying device on which the preserved vitrified germ cell is placed; a tube in which the carrying device is contained; and a cold storage device provided on an outer periphery of the tube, the tube including, inside the tube: (i) a germ cell containing region containing the carrying device, and being filled with gas and covered by the cold storage device; and (ii) a thawing solution containing region containing a thawing solution for thawing the preserved vitrified germ cell, and the carrying device coming in contact with the thawing solution in a melted state.

In the above configuration, the carrying device is covered by the cold storage device. This makes it possible to keep the germ cell, which is placed on the carrying device, in a low temperature environment for a long time. In a period in which the germ cell is kept in the low temperature environment, the thawing solution can be brought into a state suitable for thawing the germ cell. Then, the germ cell kept in the low temperature environment is immediately brought into contact with the thawing solution in the state suitable for thawing the germ cell. This makes it possible to avoid various kinds of damage (e.g., fracture damage and/or damage caused by formation of an ice crystal after devitrification) to a preserved vitrified germ cell and achieve a high survival rate when the preserved vitrified germ cell is thawed.

The device for thawing a preserved vitrified germ cell in accordance with an embodiment of the present invention is preferably configured such that: the carrying device moves toward the thawing solution in the melted state.

The configuration makes it possible to immediately and unfailingly bring a germ cell, which is kept in a low temperature environment, into contact with the thawing solution in a state suitable for thawing the germ cell.

The device for thawing a preserved vitrified germ cell in in accordance with an embodiment of the present invention is preferably configured such that: the carrying device is pushed toward the thawing solution by moving a force transmission device connected to the carrying device.

The configuration makes it possible to immediately and unfailingly bring a germ cell, which is kept in a low temperature environment, into contact with the thawing solution in a state suitable for thawing the germ cell, by use of the thawing device having a simple structure.

The device for thawing a preserved vitrified germ cell in in accordance with an embodiment of the present invention is preferably configured such that: the device for thawing is arranged such that the germ cell containing region is positioned above the thawing solution containing region, so that the carrying device falls toward the thawing solution.

The configuration makes it possible to immediately and unfailingly bring a germ cell, which is kept in a low temperature environment, into contact with the thawing solution in a state suitable for thawing the germ cell, by use of the thawing device having a simple structure.

The device for thawing a preserved vitrified germ cell in in accordance with an embodiment of the present invention is preferably configured to further include: a partition wall outside the tube, the partition wall separating an atmosphere A to which the cold storage device is exposed and an atmosphere B to which a portion of the tube is exposed, the portion of the tube being a portion where the thawing solution containing region is provided.

Since the above configuration includes the partition wall, the germ cell on the carrying device which is covered by the cold storage device is less influenced by the atmosphere B even in a case where respective conditions of the atmosphere A and the atmosphere B are different from each other. As a result, the thawing solution can be changed to a state more suitable for thawing the germ cell while the germ cell is kept in a low temperature environment.

The device for thawing a preserved vitrified germ cell in in accordance with an embodiment of the present invention is preferably configured such that: the cold storage device has a space for retaining a refrigerant.

The above configuration makes it possible to keep the temperature of the cold storage device and the temperature of the atmosphere in the vicinity of the cold storage device low for a longer time. This makes it possible, for example, to ensure a sufficient time for changing the thawing solution into a state more suitable for thawing the germ cell.

In order to solve the above problems, a method for thawing a preserved vitrified germ cell in accordance with an embodiment of the present invention includes the steps of: (a) melting a thawing solution in a frozen state in a tube, the thawing solution being used for thawing the preserved vitrified germ cell, the thawing solution being melted by warming only a thawing solution containing region between (i) a germ cell containing region and (ii) the thawing solution containing region which are provided inside the tube, (i) the germ cell containing region containing a carrying device on which the preserved vitrified germ cell is placed, and being filled with gas and covered by a cold storage device provided on a periphery of the tube, and (ii) the thawing solution containing region containing the thawing solution in the frozen state; and (b) bringing the carrying device into contact with the thawing solution which has been melted.

In the above configuration, the carrying device is covered by the cold storage device. This makes it possible to keep the germ cell, which is placed on the carrying device, in a low temperature environment for a long time. In a period in which the germ cell is kept in the low temperature environment, the thawing solution can be brought into a state suitable for thawing the germ cell. Then, the germ cell kept in the low temperature environment is immediately brought into contact with the thawing solution in the state suitable for thawing the germ cell. This makes it possible to avoid various kinds of damage (e.g., fracture damage and/or damage caused by formation of an ice crystal after devitrification) to a preserved vitrified germ cell and achieve a high survival rate when the preserved vitrified germ cell is thawed.

### Examples

### Example 1. Comparison between embodiment of invention of present application and conventional direct freezing

### 1-1. Example of thawing device in accordance with embodiment of invention of present application

### Preparation of vitrification solution

First, 700 µL of Medium-199 solution (first solution) containing 1.8 M ethylene glycol (EG), 0.3 M sucrose, and 20% (v/v) FCS was dispensed into each of wells of a 4-well petri dish. Further, 9 drops of M2 solution (second solution) containing 5M EG, 1.0M sucrose, and 4% (w/v) BSA was prepared on a petri dish. Each drop was made of 30 µL of M2 solution having the following composition. The petri dish on which the drops were placed was kept warm on a warming board at 38°C.

Composition of M2 solution (when 500 mL of M2 solution was prepared)
NaCl: 2.766 g
KCI: 0.178 g
CaCl₂·2H₂O: 0.1255 g
KH₂PO₄: 0.081 g
MgCl₂·6H₂O: 0.1525 g
Glucose: 0.5 g
HEPES: 2.4845 g
NaHCO₃: 0.175 g
Na-pyruvate: 0.018 g
Na-(lactate): 1,660 µL
Phenol Red: 250 µL
Gentamicin sulfate: 2,500 µL

The above was diluted with ultra-pure water (DW) to 500 mL in a measuring cylinder.

### Preparation of thawing solution and thawing device

First, M2 solution containing 0.3 M sucrose and 20% (v/v) FCS was prepared. This M2 solution was diluted and used as a dilute solution (thawing solution). Then, 2 mL of the dilute solution (thawing solution) was dispensed on a 35 mm dish.

The dilute solution was drawn into a tube to which a cold storage device was attached. The tube here was made of vinyl chloride, and had (i) an inner cavity whose circular cross section had a diameter of 1.8 mm to 1.9 mm and (ii) a wall whose thickness was 0.1 mm to 0.2 mm. In drawing the dilute solution, a portion inside the tube, at which portion the cold storage device was attached, was prevented from being filled with the dilute solution.

The thawing device, into which the dilute solution had been drawn, was slowly cooled in a cooling air layer at the top of liquid nitrogen, so that the dilute solution was frozen. Thereafter, the thawing device was put in liquid nitrogen, and the cold storage device was sufficiently cooled. Note that the cold storage device was made of silicon rubber or stainless steel and configured to have a shape whose inner diameter was 2 mm and outer diameter was 6 mm (wall thickness of 2 mm) and length was 30 mm in the case of silicon rubber or a shape whose inner diameter was 2 mm and outer diameter was 4 mm (wall thickness of 1 mm) and length was 30 mm in the case of stainless steel.

### Collecting embryos and balancing embryos with vitrification solution

First, bovine-derived embryos were produced by a general technique of producing in vitro fertilized eggs. Then, normality of those embryos was visually checked.

Next, 1 to 10 normal embryos, preferably 5 normal embryos were transferred by a pipette or the like into a drop of the first solution and then balanced for 2 minutes. The pipette or the like here is preferably a Pasteur pipette, but can be anything which has a thin tip and can easily form a microdroplet.

After the embryos were balanced with the first solution, the embryos were transferred into a first drop of the second solution. Immediately after the embryos were transferred into the first drop of the second solution, the embryos were transferred into a second drop of the second solution. This operation was repeated until the embryos were transferred into a seventh drop of the second solution. Subsequently, the embryos were sucked together with the second solution of the seventh drop by a pipette or the like. Then, a vitrification solution containing the embryos was thinly placed on a tip of a carrying device (made of plastic) for embryos. When the embryos were placed on the carrying device, one (1) embryo or two or more embryos can be placed.

### Preservation by vitrification

Only a portion of the carrying device, on which portion the embryos were placed, was immediately immersed in liquid nitrogen.

Next, the carrying device on which the embryos were placed was put in the thawing device which has been put in liquid nitrogen in advance. Then, the thawing device was entirely sunk in liquid nitrogen. As a result, the vitrification solution containing the embryos was frozen while the embryos were held by the carrying device and the thawing device.

Note that not more than one (1) minute was taken for a series of operations from transfer of the embryos into the second solution described above in <Collecting embryos and balancing embryos with vitrification solution> to immersion of the embryos into liquid nitrogen.

Thereafter, the thawing device containing the embryos in whole can be stored at an ultralow temperature until a time at which the embryos thus frozen is to be thawed.

### Method for thawing embryos

The thawing device including the carrying device on which the embryos frozen were placed was transferred into a container containing liquid nitrogen. Next, warm water at a temperature of 30°C was prepared. Then, on the warm water, a float plate having a hole was set afloat. The float plate was made of a material which floats on water. Examples of such a material encompass expanded resin materials such as polyurethane, polyvinyl chloride, polypropylene, urethane-based sponge, synthetic rubber, and various elastomers.

Next, the thawing device into which the carrying device had been inserted was taken out of liquid nitrogen. Then, the thawing device was inserted into the hole of the float plate and in this state where the thawing device was inserted, the thawing solution was warmed for 10 seconds. The thawing device here was inserted in the hole of the float plate until the thawing solution below the cold storage device was entirely immersed in the warm water.

After the thawing solution was warmed for 10 seconds, the carrying device, on which the embryos were placed, was pushed into the tube while the thawing device was kept immersed in the warm water. In this way, the embryos at the tip of the carrying device was immersed in the thawing solution and thawed. After the thawing device was left to stand still for one (1) minute, the carrying device was taken out of the tube.

### 1-2. Conventional direct freezing

### Preparation of freezing solution for direct freezing

First, 700 µL of M2 solution (third solution) containing 1.8 M ethylene glycol (EG), 0.4% BSA (w/v), 0.1 M sucrose, and 20% (v/v) FCS was dispensed into each of wells of a 4-well petri dish. Also, 2 mL of the M2 solution was dispensed in each of two 35 mm petri dishes. The petri dishes, on which drops of the M2 solution were placed, were kept warm on a warming board at 38°C.

The M2 solution was the M2 solution described in the above Preparation of vitrification solution.

### Collecting embryos and balancing embryos with freezing solution

First, bovine-derived embryos were produced by a general technique of producing in vitro fertilized eggs. Then, normality of those embryos was checked.

Next, 1 to 10 normal embryos, preferably 5 normal embryos were transferred by a pipette or the like to the 4-well petri dish having the wells in which the third solution were provided. After the embryos were transferred to each of all the 4 wells by transfer from one (1) well to another, the embryos were further transferred to one of the 35 mm petri dishes each containing 2 mL of the freezing solution. Then the embryos were balanced for 5 minutes. The pipette or the like here is preferably a Pasteur pipette, but can be anything which has a thin tip and can easily form a microdroplet.

After balancing with the third solution has been completed, the freezing solution containing the embryos was sucked into a tube and an inlet of the tube was closed by welding. The tube here was made of vinyl chloride, and had (i) an inner cavity whose circular cross section had a diameter of 1.8 mm to 1.9 mm and (ii) a wall whose thickness was 0.1 mm to 0.2 mm. Note that unlike a tube in an embodiment of the invention of the present application, the tube here was not provided with a cold storage device.

The tube was placed in a programmed freezer which was kept at -7°C. Then, the freezing solution was cooled from -7°C to -30°C at a very slow rate of 0.3°C/min. Thereafter, the freezing solution was put in liquid nitrogen and frozen by rapid cooling from -30°C to -196°C.

### Method for thawing embryos

The tube in liquid nitrogen was kept in the air for 6 seconds to 10 seconds (more specifically, 8 seconds), which tube held the embryos having undergone direct freezing. Then, the tube was slowly warmed. Next, the tube was put into warm water at a temperature of 30°C to 35°C and warmed for 15 seconds, so that the embryos having undergone direct freezing was thawed.

### 1-3. Test results

### In vitro culturing test of embryos which had been thawed

Each embryo having been thawed was cleaned with M2 solution seven times. Then, the embryos were cultured in an incubator which created a 5% O₂ and 5% CO₂ atmosphere at a temperature of 38.5°C. This culturing was carried out by a microdroplet method with use of an mSOF solution to which Glycin and Taurin were added.

States of growth (survival rate) of the embryos were examined 24 hours and 48 hours after the start of culturing. Note that more specifically, the survival rate of the embryos was checked by a method according to which it was observed whether or not the embryos having been thawed reformed blastoceles.

### Test result 1

Fig. 6 shows temperature changes at respective parts inside the thawing device as an Example of the invention of the present application (in a case where the material of the cold storage device was silicon rubber). As illustrated in Fig. 6, a temperature change in a germ cell containing region (air layer covered by silicon) was very small in the thawing device of the Example in accordance with the invention of the present application.

### Test result 2

Table 2 shows a test result of the survival rate of the embryos at 48 hours after the start of the culturing. It is clear from Table 2 that the survival rate of the embryos was very high in the Example of the present invention.

**Table 2**

| | Number of test embryos | Number of collected embryos | Number of survived embryos | Survival rate |
|---|---|---|---|---|
| Example of present invention | 30 | 29 | 28 | 93.3% |
| Direct freezing | 121 | 121 | 102 | 84.3% |

### Example 2. Test with regard to wall thickness of cold storage device

A temperature change in a germ cell containing region (air layer covered by silicon) was measured in each of cases using cold storage devices having respective wall thicknesses of 3 mm, 2 mm, 1.5 mm, 1 mm and 0.5 mm. Note that conditions except for the conditions described here were basically the same as those in Example 1,

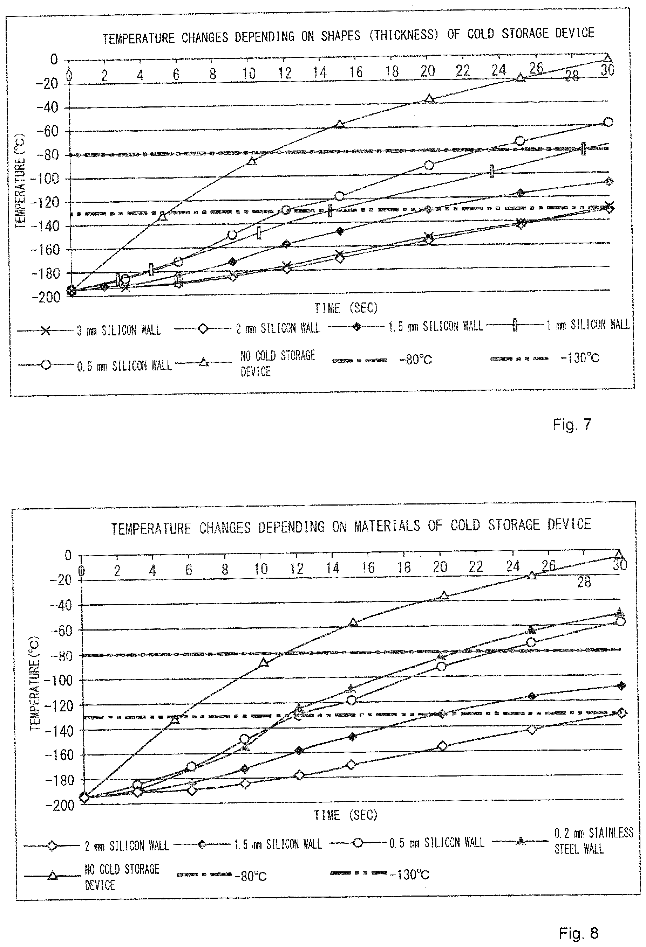

Fig. 7 shows measurement results in Example 2. It is clear from Fig. 7 that a larger wall thickness can result in a smaller temperature change of the germ cell containing region. More specifically, in cases where silicon walls having respective thicknesses of 3 mm and 2 mm were used, the temperature change of the germ cell containing region was the smallest.

### Example 3. Test with regard to material of cold storage device

A temperature change in a germ cell containing region (air layer covered by silicon or stainless steel) was measured in each of (i) cases using silicon cold storage devices having respective wall thicknesses of 0.5 mm, 1.5 mm and 2 mm and (ii) a case using a stainless steel cold storage device having a wall thickness of 0.2 mm. Note that conditions except for the conditions described here were basically the same as those in Example 1.

Fig. 8 shows measurement results in Example 3. It is clear from Fig. 8 that a temperature change in a germ cell containing region can be small in cases where (i) silicon cold storage devices having respective wall thicknesses of 0.5 mm to 2 mm are used and (ii) a stainless steel cold storage device having a wall thickness of 0.2 mm is used.

### Example 4. Test with regard to temperature changes at various positions in germ cell containing region covered by cold storage device

In the present test in Example 4, a cold storage device was made of silicon rubber and the shape of the cold storage device was arranged to have an inner diameter of 2 mm and an outer diameter of 6 mm (wall thickness of 2 mm) and a length of 30 mm. Note that conditions except for the conditions described here were basically the same as those in <Example 1 >.

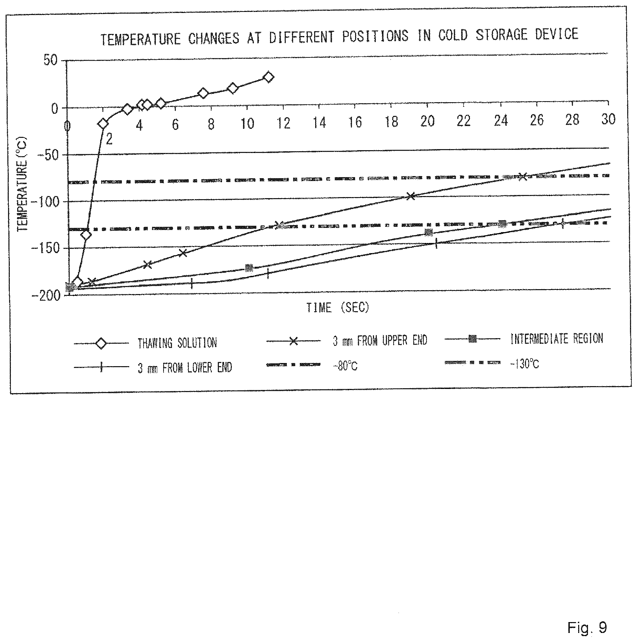

Respective temperature changes were measured in (i) a thawing solution containing region, (ii) regions 3 mm apart from respective ends of a germ cell containing region, and (iii) an intermediate region of the germ cell containing region. Fig. 9 shows measurement results. It is clear from Fig. 9 that the temperature changes were small in the regions 3 mm apart from the respective ends of the germ cell containing region and the intermediate region of the germ cell containing region. The temperature changed more slowly in the intermediate region of the germ cell containing region and the region 3 mm apart from a lower end of the germ cell containing region, as compared to the temperature change in the region 3 mm apart from an upper end of the germ cell containing region.

### Example 5. Example of cold storage device of Variation

### 5-1. Cold storage device of Variation

### Preparation of vitrification solution

A vitrification solution was prepared by a method described above in Preparation of vitrification solution of 1-1. Example of thawing device in accordance with embodiment of invention of present application.

### Preparation of thawing solution and thawing device

First, M2 solution containing 0.3 M sucrose and 20% (v/v) FCS was prepared. This M2 solution was diluted and used as a dilute solution (thawing solution). Then, 2 mL of the dilute solution (thawing solution) was dispensed on a 35 mm dish.

The dilute solution was drawn into a tube to which a cold storage device was attached. The tube here was made of vinyl chloride, and had (i) an inner cavity whose circular cross section had a diameter of 1.9 mm to 2.1 mm and (ii) a wall whose thickness was 0.05 mm to 0.15 mm. In drawing the dilute solution, a portion inside the tube, at which portion the cold storage device was attached, was prevented from being filled with the dilute solution.

The thawing device, into which the dilute solution had been drawn, was slowly cooled in a cooling air layer at the top of liquid nitrogen, so that the dilute solution was frozen. Thereafter, the thawing device was put in liquid nitrogen, and the cold storage device was sufficiently cooled. In cooling the cold storage device, the cold storage device was left to stand still in liquid nitrogen while the cold storage device was laid in a horizontal direction. This allowed liquid nitrogen to enter a refrigerant retaining region, so that the liquid nitrogen was retained in the refrigerant retaining region. Note that the cold storage device was made of silicon rubber or stainless steel and configured to have a shape whose inner diameter was 4.0 mm and outer diameter was 6.0 mm (wall thickness of 1.0 mm) and length was 30 mm in the case of silicon rubber or a shape whose inner diameter was 3.6 mm and outer diameter was 4.0 mm (wall thickness of 0.2 mm) and length was 30 mm in the case of stainless steel. Further, a cover was made of melamine sponge while a bottom portion was made of silicon. In this case, the value of "Y"' in (a) of Fig. 10 was 30 mm, the value of "X"' in (a) of Fig. 10 was 1.0 mm and the value of "Z" in (a) of Fig. 10 was 1.0 mm when the cold storage device was made of silicon rubber. Meanwhile, the value of "Y"' in (a) of Fig. 10 was 30 mm, the value of "X"' in (a) of Fig. 10 was 0.2 mm and the value of "Z" in (a) of Fig. 10 was 0.8 mm when the cold storage device was made of stainless steel.

### Collecting embryos, and balancing and preservation by vitrification of embryos with use of vitrification solution

Embryos were collected, preserved by vitrification, and thawed by methods described above in Collecting embryos and balancing embryos with vitrification solution, Preparation of vitrification solution and Preservation by vitrification of 1-1. Example of thawing device in accordance with embodiment of invention of present application.

### Method for thawing embryos

The thawing device including the carrying device on which the embryos frozen were placed was transferred into a container containing liquid nitrogen. Next, warm water at a temperature of 30°C was prepared. Then, on the warm water, a float plate having a hole was set afloat. The float plate was made of a material which floats on water. Examples of such a material encompass expanded resin materials such as polyurethane, polyvinyl chloride, polypropylene, urethane-based sponge, synthetic rubber, and various elastomers.

Next, the thawing device into which the carrying device had been inserted was taken out of liquid nitrogen. Then, the thawing device was inserted into the hole of the float plate and in this state where the thawing device was inserted, the thawing solution was warmed for 10 seconds. The thawing device here was inserted in the hole of the float plate until the thawing solution below the cold storage device was entirely immersed in the warm water.

At this time, the liquid nitrogen retained in the refrigerant retaining region kept the temperature in a germ cell containing region low, in which germ cell containing region frozen embryos were placed on a carrying device.

After the thawing solution was warmed for 10 seconds, the carrying device, on which the embryos were placed, was pushed into the tube while the thawing device was kept immersed in the warm water. In this way, the embryos at the tip of the carrying device was immersed in the thawing solution and thawed. After the thawing device was left to stand still for one (1) minute, the carrying device was taken out of the tube.

### 5-2. Case of Example in accordance with invention of present application

Preparation of a vitrification solution, a thawing solution, collecting embryos, balancing the embryos with vitrification solution, preservation by vitrification, and a method for thawing the embryos followed the procedures described in 1-1. Example of thawing device in accordance with embodiment of invention of present application.

Further, the thawing device used here was configured as described in 1-1. Example of thawing device in accordance with embodiment of invention of present application. (Specifically, the thawing device was a thawing device including a tube to which a cold storage device was attached (the tube was made of vinyl chloride, and had (i) an inner cavity whose circular cross section had a diameter of 1.8 mm to 1.9 mm and (ii) a wall whose thickness was 0.1 mm to 0.2 mm), which cold storage device was made of silicon rubber or stainless steel and configured to have a shape whose inner diameter was 2 mm and outer diameter was 6 mm (wall thickness of 2 mm) and length was 30 mm in the case of silicon rubber or a shape whose inner diameter was 2 mm and outer diameter was 4 mm (wall thickness of 1 mm) and length was 30 mm in the case of stainless steel.)

### 5-3. Conventional direct freezing

Preparation of freezing solution for direct freezing, collecting embryos, balancing the embryos with the freezing solution and a method for thawing the embryos followed the procedures described in 1-2. Conventional direct freezing.

### 5-4. Test results

### In vitro culturing test of embryos which had been thawed

Each embryo having been thawed was cleaned with M2 solution seven times. Then, the embryos were cultured in an incubator which created a 5% O₂ and 5%CO₂ atmosphere at a temperature of 38.5°C. This culturing was carried out by a microdroplet method with use of an mSOF solution to which Glycin and Taurin were added.

States of growth (survival rate) of the embryos were examined 24 hours and 48 hours after the start of culturing. Note that more specifically, the survival rate of the embryos was checked by a method according to which it was observed whether or not the embryos having been thawed reformed blastoceles.

### Test result 1

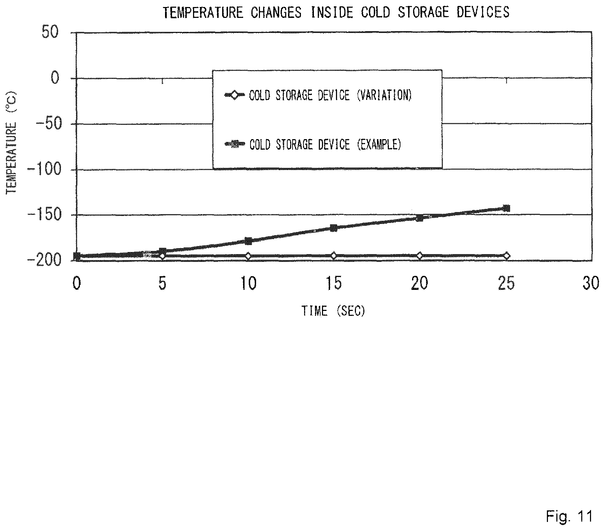

Fig. 11 shows (i) a temperature change in the germ cell containing region (air layer covered by the cold storage device) of the thawing device of a Variation (a case where the cold storage device was made of silicon rubber) in accordance with the invention of the present application and (ii) a temperature change in a germ cell containing region (air layer covered by a cold storage device) of a thawing device of an Example (a case where the cold storage device was made of silicon rubber) in accordance with the invention of the present application. As illustrated in Fig. 11, the temperature change in the germ cell containing region was smaller in the thawing device of the Variation in accordance with the invention of the present application than that of the Example in accordance with the invention of the present application.

### Test result 2

Table 3 shows a test result of the survival rate of the embryos at 48 hours after the start of the culturing. It is clear from Table 3 that the survival rate of the embryos was much higher in the case of the thawing device of the Example in accordance with the invention of the present application than that used in direct freezing. Further, the survival rate of the embryos was higher in the case of the thawing device of the Variation in accordance with the invention of the present application than that of the Example in accordance with the invention of the present application.

**Table 3**

| | Number of test embryos | Number of collected embryos | Number of survived embryos | Survival rate |
|---|---|---|---|---|
| Variation of present application | 21 | 21 | 20 | 95.2% |
| Example of present invention | 30 | 29 | 28 | 93.3% |
| Direct freezing | 121 | 121 | 102 | 84.3% |

### Test result 3

Table 4 shows a result of a test in which (i) cells constituting the embryos which have been thawed were cultured and (ii) the number of live cells and the number of dead cells were counted 48 hours after the start of culturing. It is clear from Table 4 that a proportion of live cells to all cells constituting the embryos was higher in the case of the thawing device of the Variation in accordance with the invention of the present application than that used in the Example in accordance with the invention of the present application.

The term "live cells" means living cells which are not degenerated and can undergo normal cell division. In contrast, the term "dead cells" means cells which have died and have an abnormal appearance and which cannot undergo normal cell division.

**Table 4**

| | Number of live cells | Number of dead cells | Live cell rate | n |
|---|---|---|---|---|
| Variation of present application | 108.4 | 23.9 | 81.9% | 20 |
| Examples of present invention | 76.7 | 35.2 | 68.6% | 28 |

### Industrial Applicability

An embodiment of the present invention can be widely applied to transfer of germ cells (e.g., fertilized eggs) to animals such as livestock animals etc.

### Reference Signs List

1 thawing device (device for thawing)
5 tube
6, 61 cold storage device
7 force transmission device
8 carrying device
10 germ cell containing region
11 thawing solution containing region
20 first locking device
21 second locking device
30 partition wall
40 stopper device
62 bottom portion
63 cover
64 refrigerant retaining region

## Claims

1. A device for thawing a preserved vitrified germ cell, comprising:
a carrying device on which the preserved vitrified germ cell is placed;
a tube in which the carrying device is contained; and
a cold storage device provided on an outer periphery of the tube,
the tube including, inside the tube:
(i) a germ cell containing region containing the carrying device, and being filled with gas and covered by the cold storage device; and
(ii) a thawing solution containing region containing a thawing solution for thawing the preserved vitrified germ cell, and
the carrying device coming in contact with the thawing solution in a melted state.

2. The device for thawing a preserved vitrified germ cell as set forth in claim 1, wherein:
the carrying device moves toward the thawing solution in the melted state.

3. The device for thawing a preserved vitrified germ cell as set forth in claim 2, wherein:
the carrying device is pushed toward the thawing solution by moving a force transmission device connected to the carrying device.

4. The device for thawing a preserved vitrified germ cell as set forth in claim 2, wherein:
the device for thawing is arranged such that the germ cell containing region is positioned above the thawing solution containing region, so that the carrying device falls toward the thawing solution.

5. The device for thawing a preserved vitrified germ cell as set forth in any one of claims 1 to 4, further comprising:
a partition wall outside the tube,
the partition wall separating an atmosphere A to which the cold storage device is exposed and an atmosphere B to which a portion of the tube is exposed, the portion of the tube being a portion where the thawing solution containing region is provided.

6. The device for thawing a preserved vitrified germ cell as set forth in any one of claims 1 to 5, wherein:
the cold storage device has a space for retaining a refrigerant.

7. A method for thawing a preserved vitrified germ cell, comprising the steps of:
(a) melting a thawing solution in a frozen state in a tube, the thawing solution being used for thawing the preserved vitrified germ cell, the thawing solution being melted by warming only a thawing solution containing region between (i) a germ cell containing region and (ii) the thawing solution containing region which are provided inside the tube, (i) the germ cell containing region containing a carrying device on which the preserved vitrified germ cell is placed, and being filled with gas and covered by a cold storage device provided on a periphery of the tube, and (ii) the thawing solution containing region containing the thawing solution in the frozen state; and
(b) bringing the carrying device into contact with the thawing solution which has been melted.
